Europäisches Patentamt

European Patent Office

Office européen des brevets

(19)

(11) Veröffentlichungsnummer: **0 275 339**
**A1**

(12) **EUROPÄISCHE PATENTANMELDUNG**

(21) Anmeldenummer: 87100775:3

(22) Anmeldetag: 21.01.87

(51) Int. Cl.⁴ **C07C 79/18** , C07C 76/02

(43) Veröffentlichungstag der Anmeldung:
27.07.88 Patentblatt 88/30

(84) Benannte Vertragsstaaten:
**AT BE CH DE ES FR GB GR IT LI LU NL SE**

(71) Anmelder: **Dynamit Nobel Aktiengesellschaft**
**Postfach 12 61**
**D-5210 Troisdorf(DE)**

(72) Erfinder: **Bison, Günter**
**Kleiststrasse 6**
**D-5210 Troisdorf 15(DE)**
Erfinder: **Leuck, Hans**
**Robert-Koch-Strasse 4**
**D-5210 Troisdorf 15(DE)**
Erfinder: **Thewalt, Klaus, Dr.**
**Am Tiemen 12**
**D-5810 Witten(DE)**
Erfinder: **Westermann, Hans-Jörg**
**Hitzbroicher Weg 18**
**D-5210 Troisdorf 15(DE)**

(54) **Verfahren zur Herstellung des Natriumsalzes des 2-Nitropropandiol-1,3.**

(57) Eine sichere Herstellung von Na-Nitropropandiol-1,3 aus der Reaktionslösung von Nitromethan und Paraformaldehyd durch Zugabe von Natriummethylat wird durch Verdampfungskühlung des Methanols erreicht, wobei nun Reaktionstemperaturen von 35 bis 50° C einhaltbar sind.

EP 0 275 339 A1

## Verfahren zur Herstellung des Natriumsalzes des 2-Nitropropandiol-1,3

Die Erfindung betrifft die Herstellung des Natriumsalzes des 2-Nitropropandiol-1,3, besonders des Aci-Salzes genannten Addukts von Methanol, aus methanolischen Reaktionslösungen von Nitromethan, Paraformaldehyd und Alkali durch weitere Umsetzung mit Natriummethylat.

Solche Verfahren sind bekannt, müssen jedoch bei Temperaturen zwischen 0 und 25° C unter sorgfältiger Dosierung der Ausgangsstoffe ausgeführt werden. Das Reaktionsprodukt bildet dabei eine metastabile Lösung, aus der das Aci-Salz nach Erreichen einer 5-bis 10-fachen Übersättigung schlagartig ausfällt. Dabei wird zusätzlich zu der Reaktionswärme in kurzer Zeit die erhebliche Kristallisationswärme frei und führt zu einem starken Temperaturanstieg.

Da das Aci-Salz oberhalb von 50° C zu explosionsgefährlichen Stoffen zerfallen kann, wird die Zersetzung durch kräftiges Kühlen und Unterbrechen der Zufuhr von Natriummethylat unterdrückt.

Die niedrigen Reaktionstemperaturen von höchstens 25° C erfordern aber eine Reaktionsdauer von meheren Stunden. Deswegen wird durch Überschüsse von 10 bis 20 % von Paraformaldehyd oder Natriummethylat oder beiden Stoffen versucht,die Ausbeuten zu erhöhen bzw. die Verweilzeit zu verkürzen. Diese Überschüsse gegenüber der stöchiometrischen Menge gehen jedoch verloren und erfordern zudem einen hohen Aufwand für die Reinigung, ohne daß wesentlich die Reaktionszeiten sinken, so daß der Anteil der Personalkosten an den Herstellkosten hoch wird und große Anlagen mit hohem Investionsaufwand benötigt werden.

Der Erfindung liegt daher die Aufgabe zugrunde, die Reaktionszeit des Verfahrens zu senken und nach Möglichkeit, bei gleichbleibender Ausbeute, die Menge des verbrauchten Natriummethylats und Paraformaldehyds zu verhindern.

Überraschend wurde gefunden, daß die Reaktionstemperatur erhöht werden kann, ohne daß die Bildung explosionsgefährlicher Stoffe eintritt. Im Gegensatz zu der bisherigen Reaktionsdauer von mehreren Stunden läuft überraschend dann die Reaktion in wenigen Minuten ab. Das Verfahren gemäß der Erfindung kommt dann mit wesentlich kleineren Apparaturen aus.
Allerdings ist eine genügende Sicherheit nur durch die erfindungsgemäßen Maßahmen erreichbar, durch die eine plötzliche Erhöhung der Temperatur mit großer Sicherheit vermieden wird.

Gegenstand der Erfindung ist daher ein Verfahren zur Herstellung des Natriumsalzes des 2-Nitropropandiol-1,3 durch Umsetzung von methanolischen Reaktionslösungen von Nitromethan mit Paraformaldehyd und Gehalten von Alkalien durch weitere Umsetzung mit Natriummethylat in methanolischer Lösung, dadurch gekennzeichnet, daß die Reaktionslösung und/oder die Restlösung der Herstellung des Natriumsalzes von 2-Nitropropandiol-1,3 auf dem einen Weg und Natriummethylat in methanolischer Lösung auf dem anderen Weg einem Reaktor zugeführt wird und Reaktionstemperaturen zwischen 35 und höchstens 50°C durch Verdampfungskühlung von verdampfendem Methanol durch Regelung des unterhalb Normaldruck liegenden Drucks im Reaktionsgefäß eingehalten wird.

Insbesondere wurde gefunden, daß allein verdampfendes Methanol und damit eine Verdampfungskühlung ausreicht, um mit Sicherheit Temperaturen zu vermeiden, bei denen eine Zersetzung auftritt und andererseits Temperaturen zu ermöglichen, bei denen die genannte Reaktion sehr schnell abläuft.

Zur Einstellung der jeweiligen Temperatur dient die Regelung des verminderten Drucks im Reaktionsgefäß mittels eines Druck-Konstanthalters auf solche Druckwerte, bei denen bei der jeweiligen Reaktionstemperatur ein Verdampfen des Methanols im Gefäß erfolgt. Bei konstantem Druck ist auch über die Temperatur die Reaktion steuerbar.

Wir haben gefunden, daß die Kühlung durch das Verdampfen des Methanols im Reaktionsgefäß so wirksam ist, daß plötzliche Temperaturerhöhungen vermieden werden, und Reaktionstemperaturen bis nahe an die Zersetzungstemperatur des Aci-Salzes möglich sind, im Bereich von 35 bis 50° C. Weiter haben wir gefunden, daß bei diesen Reaktionstemperaturen die Reaktionszeit auf wenige Minuten sinkt. Dadurch ist die Verweilzeit im Gefäß praktisch nur noch durch die Handhabung bedingt und nicht mehr durch die Reaktionsdauer.

Eine praktisch vollständige Ausbeute von 97,5 % oder mehr ist nunmehr erreichbar mit Überschüssen von Paraformaldehyd von nur 3 bis 10 Mol-%, vorzugsweise 4 bis 6 Mol-% über die stöchiometrisch berechnete Menge von 2 Mol Paraformaldehyd je Mol Nitromethan in der Reaktionslösung. Auch der Überschuß von Natriummethylat braucht nur noch 3 bis 10, vorzugsweise 4 bis 6 Mol-% über der stöchiometrischen Menge zu liegen.

Das Verfahrensprodukt scheidet sich aus der Reaktionslösung mit der überlegenen Reinheit von 99 % oder mehr ab, so daß eine Reinigung nicht erforderlich ist. In der verbleibenden Mutterlauge sind wenig Nebenprodukte enthalten, so daß diese erneut nach Anreicherung mit den reaktionsfähigen Stoffen bei der Reaktion eingesetzt werden kann.

Das Reaktionsgefäß ist mit einem Rührer versehen und weist einen aufgesetzten Kondensator für das verdampfte Methanol auf sowie eine Anlage zur Konstanthaltung des Vakuums.

Vorzugsweise wird Natriummethylat in methanolischer Lösung, in der üblichen Konzentration von 25 bis 35 Gew.-%, getrennt von den übrigen Reaktionsteilnehmern durch eine Pumpe eingedrückt. Bei diskontinuierlicher Ausführung der Reaktionen wird nach Ausfallen des Aci-Salzes die Suspension unter Kühlen in die Vorlage des Filters entleert. Mehrere Chargen/Std. sind erreichbar. Im gleichen Gefäß kann nach Bildung der Suspension des Aci-Salzes mit erheblichem Vorteil die Reaktion durch Zuführen weiterer Ausgangsstoffe und kontinuierlicher Abnahme des Produkts ausgeführt werden, ohne daß der Umsatzgrad sinkt. Bei den Temperaturen des erfindungsgemäßen Verfahrens ist die Reaktionsgeschwindigkeit so hoch, daß Nitromethan nicht in nennenswerter Menge mit dem verdampfenden Methanol in den Kondensator gelangt.

Die Reaktion wird über einen vorgegebenen Druck oder eine vorgegebene Temperatur gesteuert, gewünschte Temperaturänderungen können durch Soll-Wert-Einstellung des Systemdrucks vorgenommen werden. Zueinander gehörige Paare von Temperatur und Druck für Methanol sind aus Tabellen bekannt.

Der Druck während der Reaktion liegt daher zwischen 50 und 800 mb in Abhängigkeit von der jeweiligen Reaktionstemperatur.

Erfindungsgemäß wird daher insbesondere durch die Maßnahme der Verdampfungskühlung die bisher fehlende hohe Betriebssicherheit erreicht und zudem entsprechende Aufgabe eine wesentlich verkürzte Reaktionszeit bei gesteigerter Ausbeute und wesentlich verminderten Verfahrenskosten erreicht.

In den Beispielen bedeutet % stets Gew.-%.

## Beispiel 1

Ein mit Rührer und Kondensator für verdampfendes Methanol sowie einem Konstanthalter für den Druck versehenes Reaktionsgefäß wurde mit Methanol gefüllt und ein Vakuum von 395 mb eingestellt. Aus den Vorlagen wurden folgende Ströme über 3 h zudosiert.

a) 22,893 kg sog. klare Lösung, bestehend aus den Kondensationsprodukten von
2.832 g Nitromethan (95 bis 96 %)
2.982 g Paraformaldehyd (98,7%) (ca. 5,5% Überschuß)
14g KOH (30 % in $H_2O$)
17.065 g Methanol

b) 8,77 kg Natriummethylat (30 % in Methanol). Das entspricht einem Überschuß von ca. 5 % Natriummethylat.

Das Reaktionsgefäß war nicht mit Kühleinrichtungen versehen. Nach kurzer Zeit (ca. 10 min.) stieg die Temperatur auf 43,5° C und wurde dort konstant gehalten. Die gebildete Aci-Salz-Suspension gelangte über den Überlauf und eine barometrische Abtauchung in ein unter Normaldruck stehendes gekühltes Gefäß und von dort auf eine Nutsche, wo bei Raumtemperatur das Salz von der Mutterlauge getrennt wurde. Die mittleren Verweilzeiten betrugen dabei ca. 3 min. für den Reaktor I, ca. 1 min. für die Abtauchung und ca. 10 min. für den Reaktor II. Der Filterkuchen wurde unter Vakuum getrocknet.

Die trockene Auswaage betrug 8,917 kg, was einer Ausbeute von 97,5 % entspricht. Reinheit über 99,6 %.

## Beispiel 2

In einen wie in Beispiel 1 ausgerüsteten Reaktor von 2 l Inhalt wurden 300g sog. klare Lösung vorgelegt (Zusammensetzung wie Beisp. 1) und ein Vakuum von ca. 400 mb angelegt. 115 g Natriummethylat (gleicher Überschuß wie Beispiel 1) wurden in 2 min. gleichmäßig zudosiert, wobei sich nach ca. 1 min. eine Temperatur von 43° C einstellte. Nach Beenden der Zudosierung fiel die Temperatur. Der Reaktor war nicht mit einer Kühleinrichtung versehen.
Nach ca. 5 min. wurde die 34° C warme Suspension schnell auf eine Nutsche abgelassen und der Filterkuchen unter Vakuum getrocknet. Die trockene Auswaage betrug 117,2 g, was einer Ausbeute von ca. 97 % entspricht. Reinheit über 99,6 %.

## Beispiel 3 (Vergleichsbeispiel)

In einem Rührreaktor wird sog. klare Lösung mit einem Formaldehydüberschuß von ca. 12 % vorgelegt und Natriummethylat bei 20° C und einem Überschuß von ca. 14 % über 6 bis 8 h zudosiert. In dem Reaktor wurde durch Kühleinrichtungen die Temperatur auf 20° C gehalten. Nach weiteren 2 bis 4 h Nachreaktion wird abgenutscht und 3mal Methanol gewaschen.

Die Ausbeute beträgt dabei im Mittel 95 %. Reinheit unter 99 %.

Beispiel 4 (Vergleichsbeispiel)

2-Natrium-2-nitro-propandiol-1,3 ● 2 $CN_3OH$ (Aci-Salz) Einsatzmengen:

(1) 375 ml Methanol

(2) 45,8 g Nitromethan 96%ig ( = 43,97 g 100%ig) = 0,720 mol

(3) 51,0 g p-Formaldehyd = 1, 698 mol

(4) 0,3 ml KOH 33%ig; D 20°/4 = 1,319 = 0,007 mol

(5) 153,0 g Na-methylat-Lösung 30%ig = 0,85 mol

(6) 200 ml Methanol zum Waschen

Verfahren:

In einem 1 l Rührwerkskolben, ausgestattet mit Thermometer und Tropftrichter wurde (1), (2), (3) und (4) vorgelegt und 15 min. auf 65° C erhitzt, wobei die anfängliche Suspension bei Erreichen des Siedepunktes von 65° C in eine klare Lösung überging. Anschließend wurde auf 15° C abgekühlt und (5) innerhalb von 40 min. bei einer Temperatur von 15° C zugetropft. Bei dieser Temperatur wurde 1 1/2 Std. nachgerührt und der entstandene Feststoff übereine Porzellannutsche abgesaugt, mit (6) portionsweise gewaschen und getrocknet.
Ausbeute: 144,9 g farbloses Aci-Salz = 0,699 mol (97, 08%)
Reinheit: 98,5%.

Beispiel 5

Einsatzmengen:

(1) 188 ml Methanol
(2) 22,9 g Nitromethan 96%ig = 0,36 mol
(3) 23,05 g Paraformaldehyd = 0,763 mol
(4) 0,2 ml Na-methylat-Lösung 30%ig = 0,001 mol
(5) 68,10 g Na-methylat-Lösung 30%ig = 0,38 mol
(6) 100,0ml Methanol zum Waschen
Apparatur: 500 ml Rührwerkskolben, ausgestattet mit Rückflußkühler, Thermometer und Tropftrichter
Das Verfahren wurde wie in Beispiel 4 ausgeführt, jedoch wurde durch aus dem aufgesetzten Rückflußkühler zurückfließendes kondensiertes Methanol im Kolben eine Temperatur von 45° C aufrechterhalten.
Innerhalb von 8 min. wurde (5) zugegeben. Nachrührzeit 10 min.
Ausbeute: 97,8 %
Reinheit: 99,7 %.

**Ansprüche**

1. Verfahren zur Herstellung des Natriumsalzes des 2-Nitropropandiol-1,3 durch Umsetzung von methanolischen Reaktionslösungen von Nitromethan mit Paraformaldehyd und Gehalten von Alkalien durch weitere Umsetzung mit Natriummethylat in methanolischer Lösung, **dadurch gekennzeichnet,** daß die Reaktionslösung und/oder die Restlösung der Herstellung des Natriumsalzes von 2-Nitropropandiol-1,3 auf dem einen Weg und Natriummethylat in methanolischer Lösung auf dem anderen Weg einem Reaktor zugeführt wird und Reaktionstemperaturen zwischen 35 und höchstens 50° C durch Verdampfungskühlung von verdampfendem Methanol durch Regelung des unterhalb Normaldruck liegenden Drucks im Reaktionsgefäß eingehalten wird.

2. Verfahren nach Anspruch 1, dadurch gekennzeichnet, daß die Reaktionstemperatur zwischen 42 und 45° C gehalten wird.

3. Verfahren nach einem der Ansprüche 1 oder 2, dadurch gekennzeichnet, daß der Überschuß von Paraformaldehyd 3 bis 10 Mol-% beträgt.

4. Verfahren nach einem der Ansprüche 1 bis 2, dadurch gekennzeichnet, daß der Überschuß von Natriummethylat 3 bis 10 Mol-% beträgt.

# EUROPÄISCHER RECHERCHENBERICHT

Europäisches Patentamt

Nummer der Anmeldung

EP 87 10 0775

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP-A-0 071 037 (DYNAMIT NOBEL)<br>* Seite 9, Beispiel 8 *<br><br>--- | 1 | C 07 C 79/18<br>C 07 C 76/02 |
| A | US-A-4 581 178 (D. MILSTEIN)<br>* Spalte 11, Beispiele 11-24 *<br><br>--- | 1 | |
| A | DE-A-1 954 173 (HENKEL)<br>* Seiten 9,10, Beispiel 3 *<br><br>----- | 1 | |
| | | | **RECHERCHIERTE SACHGEBIETE (Int. Cl.4)**<br><br>C 07 C 79/00 |

Der vorliegende Recherchenbericht wurde für alle Patentansprüche erstellt.

| Recherchenort | Abschlußdatum der Recherche | Prüfer |
|---|---|---|
| DEN HAAG | 17-09-1987 | KINZINGER J.M. |

EPA Form 1503 03.82